# DEMANDE DE BREVET EUROPEEN

(11) **EP 4 470 522 A1**
(43) Date de publication de la demande: **04.12.2024**
(21) Numéro de dépôt: 24178321.6
(22) Date de dépôt: 27.05.2024
(51) Int. Cl.: A61K 8/27, A61K 8/36, A61K 8/37, A61Q 17/04, A61K 8/31

(54) **FORMULES SOLAIRES ANHYDRES**

(30) Priorité: 07.08.2023 FR 2308539; 26.05.2023 FR 2305301
(71) Demandeur: Hyteck, 75006 Paris (FR)
(72) Inventeur: RUBERTI, Pascale, 13122 VENTABREN (FR)
(74) Mandataire: Tripoz, Inès

(57) **Abrégé**

L'invention concerne une composition solaire exempte d'eau adaptée à un usage topique, comprenant de l'oxyde de zinc présentant une D50 supérieure ou égale à 200 nm et une D1 supérieure ou égale à 105 nm en suspension dans un véhicule lipophile cosmétiquement et/ou dermatologiquement acceptable, ladite composition solaire ne comprenant pas d'oxyde de titane.

Ladite composition est stable, c'est-à-dire que les particules d'oxyde de zinc en suspension dans le véhicule lipophile ne sédimentent pas lorsque la composition est au repos plus de 12 heures.

## Description

La présente invention se rapporte au domaine de la cosmétique et/ou de la dermatologie, et plus précisément au domaine des compositions cosmétiques et/ou dermatologiques utilisées pour la protection solaire de la peau, des muqueuses et/ou des phanères.

Les compositions solaires selon l'invention sont exemptes d'eau, cela dans le but de limiter les problèmes liés au développement microbien et à l'obligation d'utiliser des conservateurs, pouvant provoquer des réactions allergiques de l'utilisateur et ayant des effets néfastes pour l'environnement.

Dans la présente demande, une « composition exempte d'eau » désigne une composition comprenant par exemple moins de 0,5 % d'eau par rapport à la masse totale de ladite composition (c'est-à-dire moins de 0,5 g d'eau pour 100 g de composition).

Les compositions solaires selon l'invention sont stables au cours du temps.

De plus, les compositions solaires selon l'invention sont exemptes d'oxyde de titane et/ou de filtres solaires organiques synthétiques, cela dans le but de limiter leur impact sur l'environnement et/ou sur la santé des utilisateurs.

Les compositions solaires selon l'invention ne comprennent qu'un seul filtre solaire minéral qui est de l'oxyde de zinc non nanométrique d'après la règlementation européenne et le règlement cosmétique n°1223/2009. Ce règlement définit un nanomatériau comme étant un matériau insoluble ou bio-persistant, fabriqué intentionnellement et étant caractérisé par une ou plusieurs dimensions externes, ou une structure interne, sur une échelle de 1 à 100 nm.

Dont D1 supérieur à 150nm descente à 130nm

Plus précisément, l'oxyde de zinc non nanométrique selon l'invention correspond à de l'oxyde de zinc sous forme particulaire caractérisé par les deux valeurs de granulométrie suivantes :
- La granulométrie D50, qui indique que 50 % des particules de l'échantillon ont un diamètre inférieur ou égal à la valeur indiquée, par exemple une D50 de 200 nm signifie que la moitié des particules ont un diamètre supérieur ou égal à 200 nm ;
- La granulométrie D1, qui indique que 1 % des particules de l'échantillon ont un diamètre inférieur ou égal à la valeur indiquée, par exemple une D1 de 105 nm signifie que 99 % des particules ont un diamètre supérieur ou égal à 105 nm.

Dans un mode de réalisation, l'oxyde de zinc selon l'invention présente une D50 supérieure ou égale à 200 nm et une D1 supérieure ou égale à 105 nm.

Dans un mode de réalisation, l'oxyde de zinc selon l'invention présente une D50 supérieure ou égale à 200 nm et une D1 supérieure ou égale à 110 nm.

Dans un mode de réalisation, l'oxyde de zinc selon l'invention présente une D50 supérieure ou égale à 200 nm et une D1 supérieure ou égale à 120 nm.

Dans un mode de réalisation, l'oxyde de zinc selon l'invention présente une D50 supérieure ou égale à 210 nm et une D1 supérieure ou égale à 105 nm.

Dans un mode de réalisation, l'oxyde de zinc selon l'invention présente une D50 supérieure ou égale à 210 nm et une D1 supérieure ou égale à 115 nm.

Dans un mode de réalisation, l'oxyde de zinc selon l'invention présente une D50 supérieure ou égale à 210 nm et une D1 supérieure ou égale à 120 nm.

Dans un mode de réalisation, l'oxyde de zinc selon l'invention présente une D50 supérieure ou égale à 225 nm et une D1 supérieure ou égale à 105 nm.

Dans un mode de réalisation, l'oxyde de zinc selon l'invention présente une D50 supérieure ou égale à 225 nm et une D1 supérieure ou égale à 115 nm.

Dans un mode de réalisation, l'oxyde de zinc selon l'invention présente une D50 supérieure ou égale à 225 nm et une D1 supérieure ou égale à 120 nm.

Des compositions solaires exemptes d'eau ont déjà été décrites dans la littérature.

Par exemple, il est divulgué dans la demande de brevet US2012225106, déposée au nom de la société ISP INVESTMENTS INC., des compositions cosmétiques comprenant au moins un oxyde métallique, un dérivé réticulé de polyvinylpyrrolidone (PVP), et d'au moins un solvant choisi dans le groupe constitué d'alcool, d'ester, d'huile, de glycol ou une combinaison de ceux-ci. L'ajout du dérivé réticulé de PVP permet d'obtenir une composition qui ne présente pas de phénomène de sédimentation ou d'agrégation des particules métalliques. L'utilisation de ce type de polymère présente un danger pour les utilisateurs puisque l'unité monomérique de celui-ci, la 1-vinyl-2-pyrrolidone, est suspectée de provoquer le cancer. Les compositions solaires selon la présente invention ne comprennent pas ce type de PVP et/ou ses dérivés.

Il est également divulgué dans les deux demandes de brevets WO2016048274 et WO2016048425, déposées toutes les deux au nom de la société COLGATE PALMOLIVE, des compositions cosmétiques exemptes d'eau comprenant au moins un dérivé ester de type triglycéride, une première cire ayant une température de fusion comprise entre 35 et 72 °C, comme la cire d'abeille, une seconde cire présentant une température de fusion comprise entre 73 et 90 °C, et une huile végétale présentant un indice de saponification compris entre 150 et 275 mg. Ces compositions peuvent optionnellement contenir de l'oxyde de zinc et être utilisées comme des compositions solaires exemptes d'eau. Contrairement aux compositions solaires selon l'invention, la combinaison de l'oxyde de zinc et de cire présente une faible propension à permettre une dispersion correcte des particules d'oxyde métallique. De plus, les compositions solaires selon l'invention sont exemptes de cires telles que définies précédemment. Enfin, la combinaison d'acide polyhydroxystéarique, de caprilyc/capric triglycérides et de particules de Zinc non nanométrique, tel que défini dans la présente invention, permet la substitution de ces cires non-adaptées aux compositions solaires.

Le brevet américain US10470998, appartenant à la même famille que les deux demandes citées précédemment, précise l'utilisation de cire ayant une température de fusion comprise entre 35 et 72 °C, comme la cire d'abeille, dans des compositions anhydres. Ces compositions sont donc différentes des compositions de la présente invention car le véhicule lipophile cosmétiquement et/ou dermatologiquement acceptable est exempt de cires répondant à ces caractéristiques.

Il en est de même pour la demande de brevet américain US20220226210, déposée au nom de la société ZOCA LOTION LLC, qui décrit également l'incorporation de cire résistante à l'eau, telle que la cire d'abeille. Par conséquent, les compositions divulguées dans cette demande ne comprennent pas de véhicule lipophile tel que décrit dans la présente invention.

Il est également divulgué dans les deux demandes de brevet JP2018104401 et JP2022176753, déposées toutes les deux au nom de la société SAKAI CHEMICAL IND CO, des compositions permettant de disperser dans des huiles hydrocarbonées volatiles du dioxyde de titane et/ou du dioxyde de zinc, ainsi que leur utilisation possible dans des compositions solaires.

Les dispersants divulgués dans la demande, JP2018104401, sont constitués d'acide polyhydroxystéarique, de dextrane estérifié avec un acide gras et une huile hydrocarbonée volatile. Cette composition spécifique permet de disperser de façon homogène un filtre solaire constitué d'oxyde de titane, d'oxyde de zinc, et/ou un mélange des deux. Bien que ces préparations permettent de disperser des particules d'oxyde métallique, celles-ci ne permettent pas d'obtenir des compositions solaires exemptes d'eau. Les compositions des différents exemples divulgués dans cette demande sont toutes sous la forme d'émulsions. Contrairement à cette demande, le mélange selon l'invention entre des particules de zinc, de l'acide polyhydroxystéarique et des triglycérides caprilyc/capric avec les différents constituants permet d'obtenir une composition exempte d'eau stable.

Dans la seconde demande, JP2022176753, de nouveaux dispersants à base de particules d'oxyde de zinc sont présentés. Les particules sont enrobées d'un acide gras choisi dans le groupe constitué de chaine carbonée stéarique, myristique et/ou palmitique, dans des proportions molaires comprises entre 2,3 et 4,0 %mol. Le tout est en mélange avec de l'acide polyhydroxystéarique, et une huile non siliconée choisie parmi les huiles d'esters et/ou de fruits. En revanche, ces dispersants ne permettent pas d'obtenir des compositions exemptes d'eau avec des particules de zinc non nanométriques contrairement à la présente invention. De plus, il est précisé que les mélanges selon cette demande permettent la bonne dispersion de particules d'oxyde de zinc présentant une taille inférieure à 100 nm. Les exemples présentés dans ladite demande décrivent la mise en oeuvre des particules d'oxyde de zinc présentant des diamètres de 20 nm et des longueurs de 100 nm.

Il est divulgué dans la demande de brevet EP3222268, déposée au nom de la société SAKAMOTO YAKUHIN KOGYO CO, des compositions solaires comprenant des particules d'oxyde de zinc présentant un enrobage particulier, mais contrairement à la présente invention, celles-ci sont en mélange avec de l'oxyde de titane. De plus, ces particules sont dispersées dans des émulsions de type W/O.

En parallèle, la demande de brevet WO2019050785, déposée au nom de la société VIZOR LLC, divulgue différentes compositions solaires contenant de l'oxyde de zinc dispersé dans des émulsions aqueuses. Ces compositions comprenant de l'eau nécessitent l'ajout de stabilisateurs pour éviter le développement microbien et ne répondent donc pas aux caractéristiques des compositions suivant la présente invention.

De même, dans la demande WO2016121761, déposée au nom de la société SAKAI CHEMICAL IND CO, il est présenté la possibilité de disperser des oxydes métalliques nanométriques dans des huiles, en ajoutant de l'acide polyhydroxystéarique. Ces particules pouvant être de l'oxyde de zinc de taille comprise entre 10 nm à 100 nm. Enfin, ces particules sont utilisées dans des émulsions et elles peuvent être formulées en présence de dérivés siliconés. , les dérivés siliconés et l'eau, étant totalement évités dans les compositions solaires selon la présente invention puisque ces composés ne sont pas biosourcés, les compositions ci-dessus citées ne répondent donc pas aux caractéristiques des compositions selon la présente invention.

Enfin, la demande US20190183754, déposée au nom de la société AMAVARA INC., divulgue des compositions solaires comprenant des particules non-nanométriques d'oxyde de zinc en mélange avec des dérivés siliconés, comme des diméthicones et/ou des copolymères contenant des diméthicones, et parfois en mélange avec des particules d'oxyde de titane. L'utilisation de ces composés siliconés permet de disperser les particules d'oxyde de zinc, mais ces composés sont considérés comme ayant des effets délétères pour la santé des utilisateurs et/ou sur l'environnement.

De même, concernant la demande de brevet US20230157943, déposée au nom de la société EDGEWELL PERSONAL CARE BRANDS LLC, celle-ci divulgue des compositions comprenant à la fois des composés siliconés, qui sont également des diméthicones, et dans certains cas, les formulations décrites comprennent des filtres solaires organiques, tels que l'homosalate ou l'octisalate (ethylhexyl salicylate). Les silicones et les filtres solaires organiques étant totalement exclus dans les compositions solaires selon la présente invention puisque ces composés ne sont pas biosourcés, les compositions ci-dessus citées ne répondent donc pas aux caractéristiques des compositions selon la présente invention.

Les compositions solaires selon la présente invention sont exemptes de dérivés siliconés.

Une autre solution décrite dans l'art antérieur consiste à préparer des compositions solaires contenant uniquement des particules de zinc, nanométriques et non nanométriques, mais dispersées à l'aide de dérivés siliconés tels que des dérivés siloxanes (PDMS, diméthicone, etc...) ou des dérivés silanes.

C'est par exemple le cas de la demande EP3342755, dans laquelle sont divulguées des compositions solaires, sous forme d'émulsion, composées de 15 % massique d'oxyde de zinc, ayant une taille comprise entre 10 et 350 nm, et de plus de 20 % massique de composés siliconés. La présence de ce type de dérivés ne permet de revendiquer des compositions solaires certifiées BIO ; et certains de ces produits, tel que le cyclopentasiloxane (silicone D5), sont suspectés d'avoir des effets néfastes sur la santé des utilisateurs et sur l'environnement.

La demande de brevet US2009018599, déposée au nom de la société PLAYTEX PRODUCTS INC., divulgue des compositions solaires comprenant à la fois de l'oxyde de zinc et un filtre solaire organique synthétique, l'homosalate, suspecté d'être un perturbateur endocrinien. La présence de ce filtre solaire organique permet de diminuer la quantité d'oxyde de zinc mis en suspension dans les compositions. De plus, les compositions se présentent sous forme d'émulsions aqueuses. Par conséquent, les formulations divulguées dans cette demande de brevet ne peuvent pas être certifiées BIO, et celles-ci comportent des produits présentant un effet néfaste vis-à-vis de la santé des utilisateurs et/ou sur l'environnement.

Il n'existe pas, à l'heure actuelle, de composition solaire exempte d'eau, ne comprenant que de l'oxyde de zinc tel que précédemment défini, tel que précédemment défini, comme filtre solaire minéral et permettant d'obtenir une protection solaire satisfaisante supérieure ou égale à l'indice de protection SPF 30.

Dans la présente demande, un indice de protection SPF est un indice de protection qui représente le niveau de protection d'un produit solaire. Plus il est élevé, plus l'action de photoprotection est élevée. L'indice SPF mesure le pouvoir de blocage des rayons UVB, les rayons responsables principalement des coups de soleil.

Ces indices de protection sont le rapport entre le temps d'exposition nécessaire pour induire un coup de soleil avec et sans crème. Si, par exemple, une personne laisse apparaître des rougeurs au bout de 5 minutes d'exposition sans protection solaire, l'application d'une crème solaire SPF 50 permet de multiplier par 50 ce temps d'exposition avant d'avoir un coup de soleil (5 minutes * 50 = 250 minutes).

Cet indice peut être déterminé selon la norme ISO 24444:2019.

L'invention concerne une composition solaire exempte d'eau adaptée à un usage topique, comprenant de l'oxyde de zinc présentant une D50 supérieure ou égale à 200 nm et une D1 supérieure ou égale à 105 nm en suspension dans un véhicule lipophile cosmétiquement et/ou dermatologiquement acceptable, ladite composition solaire ne comprenant pas d'oxyde de titane.

Ladite composition est stable, c'est-à-dire que les particules d'oxyde de zinc en suspension dans le véhicule lipophile ne sédimentent pas lorsque la composition est au repos plus de 12 heures.

La teneur massique en oxyde de zinc dans la composition selon l'invention, est comprise dans un intervalle allant de 10 à 40 % par rapport à la masse totale de la composition solaire.

La teneur massique en oxyde de zinc dans la composition selon l'invention, est comprise dans un intervalle allant de 10 à 35 % par rapport à la masse totale de la composition solaire.

La teneur massique en oxyde de zinc dans la composition selon l'invention, est comprise dans un intervalle allant de 10 à 30 % par rapport à la masse totale de la composition solaire.

La teneur massique en oxyde de zinc dans la composition selon l'invention, est comprise dans un intervalle allant de 12,5 à 40 % par rapport à la masse totale de la composition solaire.

La teneur massique en oxyde de zinc dans la composition selon l'invention, est comprise dans un intervalle allant de 12,5 à 35 % par rapport à la masse totale de la composition solaire.

La teneur massique en oxyde de zinc dans la composition selon l'invention, est comprise dans un intervalle allant de 12,5 à 30 % par rapport à la masse totale de la composition solaire.

La teneur massique en oxyde de zinc dans la composition selon l'invention, est comprise dans un intervalle allant de 15 à 40 % par rapport à la masse totale de la composition solaire.

La teneur massique en oxyde de zinc dans la composition selon l'invention, est comprise dans un intervalle allant de 15 à 35 % par rapport à la masse totale de la composition solaire.

La teneur massique en oxyde de zinc dans la composition selon l'invention, est comprise dans un intervalle allant de 15 à 30 % par rapport à la masse totale de la composition solaire.

Dans un mode de réalisation, la teneur massique en oxyde de zinc dans la composition est comprise dans un intervalle allant de 15 à 27,5 % par rapport à la masse totale de la composition solaire.

Dans un mode de réalisation, la teneur massique en oxyde de zinc dans la composition est comprise dans un intervalle allant de 12,5 à 25 % par rapport à la masse totale de la composition solaire.

Dans un mode de réalisation, la teneur massique en oxyde de zinc dans la composition est comprise dans un intervalle allant de 12,5 à 27,5 % par rapport à la masse totale de la composition solaire.

Dans un mode de réalisation, la teneur massique en oxyde de zinc dans la composition est comprise dans un intervalle allant de 15 à 25 % par rapport à la masse totale de la composition solaire.

Dans un mode de réalisation, la composition solaire selon la présente invention est exempte de filtres solaires organiques synthétiques.

Dans la présente invention, on entend par filtres solaires organiques synthétiques par exemple les composés décrits dans l'ouvrage « Conception des produits cosmétiques : la formulation », d'Anne-Marie PENSE-LHERITIER 2° édition, publié en 2018 chez COSMETIC VALLEY EDITIONS, et classés dans les groupes suivants :
- Dérivés benzophénones : benzophénone 3 (BP3), benzophénones 4 et 5 (BP4/5), diethylamino hydroxybenzoyl hexyl benzoate (DHHB) ;
- Dérivés du camphre : 3-benzylidene camphor (3BC), 4-methylbenzylidene camphor (4MBC), camphor benzalkonium methosulfate, polyacrylamidométhyl benzylidène camphor, benzylidene camphor sulfonic acid, terephtalydene dicamphor sulfonic acid (TDSA) ;
- Dérivés de l'acide salicylique : homosalate (HMS), ethylhexyl salicylate (EHS) ;
- Dérivés benzimidazoles : phenylbenzimidazole sulfonic acid (PBSA), disodium phenyl dibenzimidazole tetrasulfonate (DPDT) ;
- Dérivés benzotriazoles : drométrizole trisiloxane (DTS), methylene bis-benzotriazolyl tetramethylbutylphenol (MBBT) ;
- Dérivés benzoyl méthane : butyl méthoxydibenzoylméthane (BMDBM) ;
- Dérivés de l'acide 4-aminobenzoïque : acide 4-aminobenzoïque (PABA), diméthyl PABA (OD-PABA) ;
- Dérivés Cinnamates 3 : ethylhexyl methoxycinnamate (EHMC ou OMC), isoamyl *p*-methoxycinnamate (IMC), octocrylene (OCR) ;
- Dérivés triazines : ethylhexyl triazone (EHT), diethylhexyl butamido triazone, méthylène bis-benzotriazolyl tétraméthylbutylphénol (MBBT) ;
- Dérivés malonates : polysilicone-15.

Dans un mode de réalisation, la composition solaire selon l'invention est exempte de polyvinylpyrrolidone et/ou de ses dérivés.

Les compositions solaires selon la présente invention sont exemptes de dérivés siliconés.

Dans un mode de réalisation, la composition solaire selon l'invention est exempte de composés siliconés volatiles ou non volatiles d'après la classification publiée par l'observatoire des cosmétiques en novembre 2016.

Dans la présente invention, on entend par composés siliconés volatiles des composés siloxanes cycliques ou linéaires de faible poids moléculaire. Les principaux composés siliconés volatiles répondent aux dénominations INCI suivantes : cyclomethicone, cyclopentasiloxane, trisiloxane et/ou diméthicone.

Dans la présente invention, on entend par composés siliconés non volatiles des composés siloxanes linéaires de poids moléculaire moyen et/ou de haut poids moléculaire, ainsi que les composés de types polydiméthylsiloxane de poids moléculaire moyen et/ou de haut poids moléculaire. Les principaux composés siliconés non volatiles de poids moléculaire moyen répondent aux dénominations INCI suivantes : diméthicone, aminodiméthicone, polysiloxane. Les principaux composés siliconés non volatiles de poids moléculaire moyen répondent aux dénominations INCI suivantes : diméthicone, diméthiconol.

Dans un mode de réalisation, la composition solaire selon l'invention est exempte de dérivés siliconés tels que des diméthicones, des dérivés siloxanes (PDMS, diméthicone, dimethiconol) et/ou des dérivés silanes.

Dans un mode de réalisation, la composition solaire selon l'invention est exempte de composé polydiméthylsiloxane (PDMS).

Dans un mode de réalisation, la composition solaire selon l'invention est exempte de diméthicone.

Dans un mode de réalisation, la composition solaire selon l'invention est exempte de diméthiconol.

Dans un mode de réalisation, la composition solaire selon l'invention est exempte de cire ayant une température de fusion comprise dans un intervalle allant de 35 à 72 °C.

Dans un mode de réalisation, la composition solaire selon l'invention est exempte de cire d'abeille.

Dans un mode de réalisation, la composition solaire selon l'invention est exempte de cire végétale.

Dans un mode de réalisation, la composition solaire selon l'invention comprend uniquement des composés biosourcés.

Dans un mode de réalisation, la composition solaire selon l'invention présente un indice de protection SPF supérieur ou égale à 20.

Dans un mode de réalisation, la composition solaire selon l'invention présente un indice de protection SPF supérieur ou égale à 25.

Dans un mode de réalisation, la composition solaire selon l'invention présente un indice de protection SPF supérieur ou égale à 30.

Dans un mode de réalisation, la composition solaire selon l'invention présente un indice de protection SPF compris dans un intervalle allant de 20 à 50.

Dans un mode de réalisation, la composition solaire selon l'invention présente un indice de protection SPF compris dans un intervalle allant de 25 à 50.

Dans un mode de réalisation, la composition solaire selon l'invention présente un indice de protection SPF compris dans un intervalle allant de 30 à 50.

Dans un mode de réalisation, la composition solaire selon l'invention, est caractérisée en ce que le véhicule lipophile cosmétiquement et/ou dermatologiquement acceptable comprend au moins un excipient choisi dans le groupe comprenant le caprilyc/capric triglycéride, l'acide polyhydroxystéarique.

Dans un mode de réalisation, la teneur massique en caprilyc/capric triglycéride est comprise dans un intervalle allant de 15 à 50 % par rapport à la masse totale de la composition solaire.

Dans un mode de réalisation, la teneur massique en caprilyc/capric triglycéride est comprise dans un intervalle allant de 15 à 45 % par rapport à la masse totale de la composition solaire. Dans un mode de réalisation, la teneur massique en caprilyc/capric triglycéride est comprise dans un intervalle allant de 15 à 40 % par rapport à la masse totale de la composition solaire.

Dans un mode de réalisation, la teneur massique en caprilyc/capric triglycéride est comprise dans un intervalle allant de 15 à 35 % par rapport à la masse totale de la composition solaire.

Dans un mode de réalisation, la teneur massique en caprilyc/capric triglycéride est comprise dans un intervalle allant de 19 à 35 % par rapport à la masse totale de la composition solaire.

Dans un mode de réalisation, la teneur massique en caprilyc/capric triglycéride est comprise dans un intervalle allant de 20 à 35 % par rapport à la masse totale de la composition solaire.

Dans un mode de réalisation, la teneur massique en acide polyhydroxystéarique est comprise dans un intervalle allant de 0,2 à 5 % par rapport à la masse totale de la composition solaire.

Dans un mode de réalisation, la teneur massique en acide polyhydroxystéarique est comprise dans un intervalle allant de 0,25 à 5 % par rapport à la masse totale de la composition solaire.

Dans un mode de réalisation, la teneur massique en acide polyhydroxystéarique est comprise dans un intervalle allant de 0,5 à 5 % par rapport à la masse totale de la composition solaire.

Dans un mode de réalisation, la teneur massique en acide polyhydroxystéarique est comprise dans un intervalle allant de 1 à 5 % par rapport à la masse totale de la composition solaire.

Dans un mode de réalisation, la composition solaire selon l'invention, est caractérisée en ce que le véhicule lipophile cosmétiquement et/ou dermatologiquement acceptable comprend en outre un excipient choisi dans le groupe des alcanes.

On appelle « alcane » un hydrocarbure saturé étant constitué uniquement d'atomes de carbone et d'hydrogènes liés entre eux par des liaisons covalentes simples dont la formule générale est CₙH₂ₙ₊₂.

On appelle « alcane linéaire » un alcane dans lequel chaque atome de carbone est lié au maximum à deux atomes de carbone.

On appelle « alcane ramifié » un alcane dans lequel certains atomes de carbone sont liés à trois, voire quatre atomes de carbone.

On appelle « alcane en Cₓ » un alcane constitué de x atomes de carbone, ledit alcane pouvant être linéaire ou ramifié. Par exemple, le dodécane est un alcane en C₁₂.

Dans un mode de réalisation, la formulation solaire selon l'invention comprend au moins un alcane pouvant être linéaire ou ramifié en C₈-C₁₆.

Dans un mode de réalisation, la formulation solaire selon l'invention comprend au moins un alcane pouvant être linéaire en C₈-C₁₆.

Dans un mode de réalisation, la teneur massique en alcane est comprise dans un intervalle allant de 1 à 25 % par rapport à la masse totale de la composition solaire.

Dans un mode de réalisation, la teneur massique en alcane est comprise dans un intervalle allant de 1 à 20 % par rapport à la masse totale de la composition solaire.

Dans un mode de réalisation, la teneur massique en alcane est comprise dans un intervalle allant de 1 à 17,5 % par rapport à la masse totale de la composition solaire.

Dans un mode de réalisation, la teneur massique en alcane est comprise dans un intervalle allant de 1 à 5 % par rapport à la masse totale de la composition solaire

Dans un mode de réalisation, la formulation solaire selon l'invention comprend un moins un alcane étant le dodécane. masse totale de

Dans un mode de réalisation, l'excipient est choisi dans le groupe des alcanes est du dodécane.

Dans un mode de réalisation, la teneur massique en dodécane est comprise dans un intervalle allant de 1 à 25 % par rapport à la masse totale de la composition solaire.

Dans un mode de réalisation, la teneur massique en dodécane est comprise dans un intervalle allant de 1 à 20 % par rapport à la masse totale de la composition solaire.

Dans un mode de réalisation, la teneur massique en dodécane est comprise dans un intervalle allant de 1 à 17,5 % par rapport à la masse totale de la composition solaire.

Dans un mode de réalisation, la teneur massique en dodécane est comprise dans un intervalle allant de 1 à 5 % par rapport à la masse totale de la composition solaire.

Dans un mode de réalisation, la formulation solaire selon l'invention comprend au moins un émollient choisi dans le groupe constitué des monoesters d'acides carboxyliques gras, saturés ou insaturés, comprenant de 2 à 30 atomes de carbone, pouvant porter une fonction alcool, avec des alcools portant de 2 à 30 atomes de carbone, linéaires, branchés, saturés ou insaturés.

Dans un mode de réalisation, l'émollient de type monoester est choisie dans le groupe constitué par l'isoamyle laurate, la coco-caprylate, le 2-éthylhexanoate de cétyle, le 2-hexyldécylstearate, le 2-hexyldécyllaurate, le néopentanoate d'isodécyle, l'isononanoate d'isononyle, le palmitate de 2-éthylhexyle, le stéarate de 2-éthylhexyle, le myristate d'isopropyle, le palmitate d'isopropyle, le stéarate d'isopropyle, l'isostéarate d'isopropyle, l'oléate d'isopropyle, le stéarate de butyle, le stéarate d'isooctyle, le stéarate d'octyle (huile de purcellin), le stéréate d'isononyle, le stéarate d'isocétyle, l'isononanoate d'isononyle, l'isononanoate d'isotridécyle, l'isononanoate de cétéaryle, le laurate d'hexyle, le laurate de 2-éthylhexyle, l'isostéarate de 2-éthylhexyle, le myristate de 2-octyldécyle, le néopentanoate d'isostéaryle, le néopentanoate de tridécyle, le cocoate de 2-éthylhexyle, le palmitate de 2-octyldodécyle, l'acide butyloctanoïque-2-butyloctanoate, l'acétate de diisotridecyle, le stéarate de n-butyle, le laurate de n-hexyle, l'oléate de n-décyle, l'oléate d'oléyle, l'érucate d'oléyle, l'oléate d'érucyle et l'érucate d'érucyle, seuls ou en mélange.

Dans un mode de réalisation, la formulation solaire selon l'invention comprend de l'isoamyle laurate et/ou du coco-caprylate.

Dans un mode de réalisation, la formulation solaire selon l'invention comprend de l'isoamyle laurate.

Dans un mode de réalisation, la teneur massique en isoamyle laurate est comprise dans un intervalle allant de 0 à 15 % par rapport à la masse totale de la composition solaire.

Dans un mode de réalisation, la teneur massique en isoamyle laurate est comprise dans un intervalle allant de 0 à 12,5 % par rapport à la masse totale de la composition solaire.

Dans un mode de réalisation, la teneur massique en isoamyle laurate est comprise dans un intervalle allant de 0 à 10 % par rapport à la masse totale de la composition solaire.

Dans un mode de réalisation, la teneur massique en isoamyle laurate est comprise dans un intervalle allant de 1 à 15 % par rapport à la masse totale de la composition solaire.

Dans un mode de réalisation, la teneur massique en isoamyle laurate est comprise dans un intervalle allant de 1 à 12,5 % par rapport à la masse totale de la composition solaire.

Dans un mode de réalisation, la teneur massique en isoamyle laurate est comprise dans un intervalle allant de 1 à 10 % par rapport à la masse totale de la composition solaire.

Dans un mode de réalisation, la teneur massique en isoamyle laurate est comprise dans un intervalle allant de 5 à 15 % par rapport à la masse totale de la composition solaire.

Dans un mode de réalisation, la teneur massique en isoamyle laurate est comprise dans un intervalle allant de 5 à 12,5 % par rapport à la masse totale de la composition solaire.

Dans un mode de réalisation, la teneur massique en isoamyle laurate est comprise dans un intervalle allant de 5 à 10 % par rapport à la masse totale de la composition solaire.

Dans un mode de réalisation, la formulation solaire selon l'invention comprend du coco-caprylate.

Dans un mode de réalisation, la teneur massique en coco-caprylate est comprise dans un intervalle allant de 0 à 10 % par rapport à la masse totale de la composition solaire.

Dans un mode de réalisation, la teneur massique en coco-caprylate est comprise dans un intervalle allant de 0 à 7,5 % par rapport à la masse totale de la composition solaire.

Dans un mode de réalisation, la teneur massique en coco-caprylate est comprise dans un intervalle allant de 0 à 5 % par rapport à la masse totale de la composition solaire.

Dans un mode de réalisation, la teneur massique en coco-caprylate est comprise dans un intervalle allant de 1 à 10 % par rapport à la masse totale de la composition solaire.

Dans un mode de réalisation, la teneur massique en coco-caprylate est comprise dans un intervalle allant de 1 à 7,5 % par rapport à la masse totale de la composition solaire.

Dans un mode de réalisation, la teneur massique en coco-caprylate est comprise dans un intervalle allant de 1 à 5 % par rapport à la masse totale de la composition solaire.

Dans un mode de réalisation, la teneur massique en coco-caprylate est comprise dans un intervalle allant de 2 à 8 % par rapport à la masse totale de la composition solaire.

Dans un mode de réalisation, la teneur massique en coco-caprylate est comprise dans un intervalle allant de 2 à 5 % par rapport à la masse totale de la composition solaire.

Dans un mode de réalisation, la formulation solaire selon l'invention comprend au moins un agent fonctionnel choisi dans le groupe constitué par les esters de glycérol d'acides gras.

Dans un mode de réalisation, la formulation solaire selon l'invention est caractérisée en ce qu'elle comprend en outre au moins un agent fonctionnel choisi dans le groupe constitué des alcools gras, des sels d'acides gras, composés dérivés d'acides gras, lecithin, xanthan gum, glycerin, Stearamidopropyl Dimethylamine, seuls ou en combinaison.

Dans un mode de réalisation, la formulation solaire selon l'invention est caractérisée en ce qu'elle comprend en outre au moins une huile végétale.

Dans un mode de réalisation, la formulation solaire selon l'invention est caractérisée en ce que ladite au moins une huile végétale est extraite de toute partie de l'espèce végétale choisie par exemple dans le groupe constitué de Passiflora Edulis Seed, Sesamum Indicum Seed, Cocos nucifera, Carthamus Tinctorius Seed, Helianthus Annuus Seed, Cyperus esculentus, Punica granatum, Prunus armeniaca, Crambe abyssinia, Euterpe oleracea, Prunus dulcis, Carapa guaianensis, Argania spinosa, Hippophae rhamindes, Persea gratissima, Avena Sativa, Orbignya oleifera, Adansonia digitata, Mauritia flexuosa, Camelia sinensis, Camelina sativa, Carthamus tinctorius, Ribes nigrum, Prunus Cerasus, Cannabis sativa, Cucumis sativis, Gossypium herbaceum, Vaccinium macrocarpon, Balanites roxburghii, Opuntia ficus indica, Rubus idaeus, Passiflora incarnata, Triticum vulgare, Hibiscus sabdariffa, Plukenetia volubilis, Simmondsia chinensis, Pongamia glabra, Aleurites moluccana, Macadamia integrifolia, Sclerocarya birrea, Citrullus vulgaris, Corylus avellana, Bertholletia excelsa, Butyrospermum parkii, Olea europaea, Oenothera biennis, Elaeis guineensis, Vitis vinifera, Perilla frutescens, Caryocar coriaceum, Pentaclethra macroloba, Ricinus communis, Rosa rubiginosa, Pouteria sapota, Oryza sativa, Solanum lycopersicum, Calodendrum capense, seules ou en combinaison.

Dans un mode de réalisation, la formulation solaire selon l'invention est caractérisée en ce que ladite au moins une huile végétale est extraite de toute partie de l'espèce végétale choisie dans le groupe constitué de Passiflora Edulis Seed, Sesamum Indicum Seed, Cocos nucifera, Carthamus Tinctorius Seed, Helianthus Annuus Seed, seules ou en combinaison.

Dans un mode de réalisation, la formulation solaire selon l'invention est caractérisée en ce qu'elle comprend un mélange d'huiles végétales extraite de toute partie des espèces végétales choisies dans le groupe constitué de Passiflora Edulis Seed, Sesamum Indicum Seed et/ou Helianthus Annuus Seed.

Dans un mode de réalisation, la formulation solaire selon l'invention est caractérisée en ce qu'elle comprend un mélange d'huiles végétales extraites de toute partie des espèces végétales choisies dans le groupe constitué de Passiflora Edulis Seed, Carthamus Tinctorius Seed, Helianthus Annuus Seed et/ou Cocos nucifera.

Dans un mode de réalisation, la formulation solaire selon l'invention est caractérisée en ce qu'elle comprend un mélange d'huiles végétales extraites de toute partie des espèces végétales choisies dans le groupe constitué de Passiflora Edulis Seed, Carthamus Tinctorius Seed, Helianthus Annuus Seed et/ou Cocos nucifera.

Dans un mode de réalisation, la formulation solaire selon l'invention est caractérisée en ce qu'elle comprend en outre au moins un agent fonctionnel choisi par exemple dans le groupe constitué de composés dérivés d'acides gras suivants : acide laurique, acide myristique, acide palmitique, acide stéarique, acide polyhydroxystéarique, acide isostéarique, seuls ou en combinaison.

Dans un mode de réalisation, la formulation solaire selon l'invention est caractérisée en ce qu'elle comprend en outre au moins un agent fonctionnel choisi dans le groupe constitué des alcools gras suivant : alcool laurylique, alcool cétylique, alcool stéarylique, hexyl dodécanol, and alcool isostéarylique, seuls ou en combinaison.

Dans un mode de réalisation, la formulation solaire selon l'invention est caractérisée en ce qu'elle comprend en outre un agent fonctionnel choisi dans le groupe constitué de sels d'acides gras ou de composés dérivés d'acides gras choisis dans le groupe constitué du Glycéryl stéarate, Glycéryl stéarate SE, Sorbitan Olivate, Glycéryl Stéarate Citrate, Polyglyceryl-3 Stéarate, Polyglyceryl-3 Cocoate, Polyglyceryl-4 Caprate, Polyglyceryl-4 Cocoate, Polyglyceryl-6 Caprylate, Polyglyceryl-6 Ricinoleate, Polyglyceryl-3 Polyricinoleate, Glycéryl Oléate, Glycéryl oléate Citrate, Glycéryl Citrate, Lactate, Linoléate, Oléate, Sucrose Laurate, Sucrose Stéarate, Sucrose laurate, Polyglyceryl-6 Stéarate, Polyglyceryl-6 Behenate, polysorbate 80, Méthyle glucose sesquistearate, Cétéaryle olivate, seuls ou en combinaison.

Dans un mode de réalisation, la formulation solaire selon l'invention comprend au moins un agent fonctionnel choisi dans le groupe constitué par les monoesters d'acides gras en C₁₂-C₂₂ avec du glycérol.

Dans un mode de réalisation, la formulation solaire selon l'invention comprend au moins un agent fonctionnel choisi dans le groupe constitué par le monomyristate de glycéryle, le palmitate de glycéryle, le stéarate de glycéryle, l'isostéarate de glycéryle, l'oléate de glycéryle, le dioléate de glycéryle, le distéarate de glycéryle, le laurate de glycéryle, le trilaurate de glycéryle, le triarachidine, le tribéhénine, le tricaprine, le tricapryline, le triglycéride caprylique/caprique, le triérucine, le triheptanoïne, le triheptylundécanoine, le triisononanoin, le triisopalmitine, le triisostéarine, le trilinoléine, le trimyristine, le trioctanoïne, le trioléine, le tripalmitine, le tripalmitoléine, le triricinoléine, le tristéarine et le triundécanoine.

Dans un mode de réalisation, la composition pour le traitement de fibres kératiniques selon l'invention comprend au moins un agent fonctionnel choisi parmi les esters de glycérol oligomériques.

Dans un mode de réalisation, la formulation solaire selon l'invention est caractérisée en ce qu'elle comprend au moins un polymère et/ou un copolymère filmogène.

Au sens de la présente invention, on entend par polymère filmogène ou par copolymère filmogène un polymère ou un copolymère apte à former un film macroscopiquement continu notamment sur la peau dans le cadre de notre invention.

Dans un mode de réalisation, la formulation solaire selon l'invention est caractérisée en ce que le polymère filmogène est choisi dans le groupe comprenant : les polyesters, les polyamides et/ou les polyuréthanes.

Dans un mode de réalisation, la formulation solaire selon l'invention comprend en outre, un copolymère filmogène.

Dans un mode de réalisation, la teneur massique en copolymère filmogène est comprise dans un intervalle allant de 0 à 15 % par rapport à la masse totale de la composition solaire.

Dans un mode de réalisation, la teneur massique en copolymère filmogène est comprise dans un intervalle allant de 0 à 12,5 % par rapport à la masse totale de la composition solaire.

Dans un mode de réalisation, la teneur massique en copolymère filmogène est comprise dans un intervalle allant de 0 à 10 % par rapport à la masse totale de la composition solaire.

Dans un mode de réalisation, la teneur massique en copolymère filmogène est comprise dans un intervalle allant de 0 à 8,5 % par rapport à la masse totale de la composition solaire.

Dans un mode de réalisation, la teneur massique en copolymère filmogène est comprise dans un intervalle allant de 0 à 7 % par rapport à la masse totale de la composition solaire.

Dans un mode de réalisation, la teneur massique en copolymère filmogène est comprise dans un intervalle allant de 1 à 15 % par rapport à la masse totale de la composition solaire.

Dans un mode de réalisation, la teneur massique en copolymère filmogène est comprise dans un intervalle allant de 1 à 12,5 % par rapport à la masse totale de la composition solaire.

Dans un mode de réalisation, la teneur massique en copolymère filmogène est comprise dans un intervalle allant de 1 à 10 % par rapport à la masse totale de la composition solaire.

Dans un mode de réalisation, la teneur massique en copolymère filmogène est comprise dans un intervalle allant de 1 à 8,5 % par rapport à la masse totale de la composition solaire.

Dans un mode de réalisation, la teneur massique en copolymère filmogène est comprise dans un intervalle allant de 1 à 7 % par rapport à la masse totale de la composition solaire.

Dans un mode de réalisation, ce copolymère filmogène est le Capryloyl Glycerin/Sebacic Acid Copolymer. Ce copolymère présente également l'avantage d'améliorer la protection solaire de la formulation solaire selon l'invention.

Dans un mode de réalisation, la teneur massique en Capryloyl Glycerin/Sebacic Acid Copolymer est comprise dans un intervalle allant de 0 à 15 % par rapport à la masse totale de la composition solaire.

Dans un mode de réalisation, la teneur massique en Capryloyl Glycerin/Sebacic Acid Copolymer est comprise dans un intervalle allant de 0 à 12,5 % par rapport à la masse totale de la composition solaire.

Dans un mode de réalisation, la teneur massique en Capryloyl Glycerin/Sebacic Acid Copolymer est comprise dans un intervalle allant de 0 à 10 % par rapport à la masse totale de la composition solaire.

Dans un mode de réalisation, la teneur massique en Capryloyl Glycerin/Sebacic Acid Copolymer est comprise dans un intervalle allant de 0 à 8,5 % par rapport à la masse totale de la composition solaire.

Dans un mode de réalisation, la teneur massique en Capryloyl Glycerin/Sebacic Acid Copolymer est comprise dans un intervalle allant de 0 à 7 % par rapport à la masse totale de la composition solaire.

Dans un mode de réalisation, la teneur massique en Capryloyl Glycerin/Sebacic Acid Copolymer est comprise dans un intervalle allant de 1 à 15 % par rapport à la masse totale de la composition solaire.

Dans un mode de réalisation, la teneur massique en Capryloyl Glycerin/Sebacic Acid Copolymer est comprise dans un intervalle allant de 1 à 12,5 % par rapport à la masse totale de la composition solaire.

un mode de réalisation, la teneur massique en Capryloyl Glycerin/Sebacic Acid Copolymer est comprise dans un intervalle allant de 1 à 10 % par rapport à la masse totale de la composition solaire.

Dans un mode de réalisation, la teneur massique en Capryloyl Glycerin/Sebacic Acid Copolymer est comprise dans un intervalle allant de 1 à 8,5 % par rapport à la masse totale de la composition solaire.

un mode de réalisation, la teneur massique en Capryloyl Glycerin/Sebacic Acid Copolymer est comprise dans un intervalle allant de 1 à 7 % par rapport à la masse totale de la composition solaire.

Dans un mode de réalisation, la formulation solaire selon l'invention comprend en outre au moins un agent gélifiant.

Dans un mode de réalisation, la formulation solaire selon l'invention comprend en outre au moins un agent gélifiant choisi parmi le groupe comprenant les gélifiants organiques, les gélifiants polymériques, et/ou leurs mélanges.

Dans un mode de réalisation, la formulation solaire selon l'invention comprend en outre au moins un agent gélifiant d'origine végétale.

Dans un mode de réalisation, la formulation solaire selon l'invention est caractérisée en ce que le gélifiant polymérique est choisi dans le groupe comprenant : les polyesters et leurs dérivés, les dérivés de polyamides, les polyuréthanes et/ou leurs mélanges.

Dans un mode de réalisation, la teneur massique en gélifiant polymérique est comprise dans un intervalle allant de 0 à 10 % par rapport à la masse totale de la composition solaire.

Dans un mode de réalisation, la teneur massique en gélifiant polymérique est comprise dans un intervalle allant de 0 à 8 % par rapport à la masse totale de la composition solaire.

Dans un mode de réalisation, la teneur massique en gélifiant polymérique est comprise dans un intervalle allant de 0 à 6 % par rapport à la masse totale de la composition solaire.

Dans un mode de réalisation, la teneur massique en gélifiant polymérique est comprise dans un intervalle allant de 1 à 10 % par rapport à la masse totale de la composition solaire.

Dans un mode de réalisation, la teneur massique en gélifiant polymérique est comprise dans un intervalle allant de 1 à 8 % par rapport à la masse totale de la composition solaire.

Dans un mode de réalisation, la teneur massique en gélifiant polymérique est comprise dans un intervalle allant de 1 à 6 % par rapport à la masse totale de la composition solaire.

Dans un mode de réalisation, la teneur massique en gélifiant polymérique est comprise dans un intervalle allant de 2 à 10 % par rapport à la masse totale de la composition solaire.

Dans un mode de réalisation, la teneur massique en gélifiant polymérique est comprise dans un intervalle allant de 2 à 8 % par rapport à la masse totale de la composition solaire.

Dans un mode de réalisation, la teneur massique en gélifiant polymérique est comprise dans un intervalle allant de 2 à 6 % par rapport à la masse totale de la composition solaire.

Dans un mode de réalisation, la formulation solaire selon l'invention est caractérisée en ce que le gélifiant polyester est choisi dans le groupe comprenant les polyesters issus de la réaction d'estérification entre les dérivés de polyglycérols suivant : diglycérol, triglycérol, tetraglycérol, pentaglycérol, hexaglycérol, heptaglycérol, octaglycérol, nonaglycérol, décaglycérol, hexadecaglycérol, octadécaglycérol, et/ou eicosaglycérol ; et les dérivés monoacides carboxylique suivant acide acétique, acide propionique, acide butyrique, acide valérique, acide caprique, acide caprylique, acide 2-ethylhexanoic, acide isononanique, acide capric, acide laurique, acide myristique, acide isomyristique, acide palmitique, acide isopalmitique, acide stéarique, acide isostéarique, acide ricinoléique, acide oléique, acide linoléique, acide linolénique, acide arachidique, acide isoarachidique, acide béhénique, et/ou acide érucique.

Dans un mode de réalisation, la formulation solaire selon l'invention est caractérisée en ce que le gélifiant polyester est choisi dans le groupe comprenant les polyesters issus de la réaction d'estérification entre les dérivés de polyglycérols suivant : diglycérol, triglycérol, tetraglycerol, pentaglycerol, hexaglycérol, heptaglycérol, octaglycerol, nonaglycerol, décaglycérol, hexadécaglycerol, octadécaglycérol, and eicosaglycérol et les dérivés diacides carboxyliques suivant : acide oxalique, acide malonique, acide succinique, acide glutarique, acide adipique, acide pimélique, acide subérique, acide azélaique, acide sébacique, acide dimethyloctadecanedioique, acide eicosanedioique, acide, phtalique, acide isophthalique, acide téréphtalique, et/ou acide maléique.

Dans un mode de réalisation, la formulation solaire selon l'invention est caractérisée en ce que le gélifiant polyester est choisi dans le groupe comprenant le trihydroxystéarin, PLANDOOL^{™} G, PLANDOOL^{™} H, LUSPLAN^{™} DD-DA5 et/ou LUSPLAN^{™} DD-DA7.

Dans un mode de réalisation, la formulation solaire selon l'invention est caractérisée en ce que le gélifiant polyuréthane est choisi dans le groupe des gélifiants polyuréthane tels que ceux décrits dans le brevet EP3606973.

Dans un mode de réalisation, la formulation solaire selon l'invention est caractérisée en ce que le gélifiant polyamide est un dérivé polyamide soluble dans les huiles et apte à gélifier, tout en fournissant des gels ayant une texture agréable au toucher même avec une quantité limitée de gélifiant.

Dans un mode de réalisation, la formulation solaire selon l'invention est caractérisée en ce que le gélifiant polyamide est choisi parmi les gélifiants polyamides décrits dans le brevet EP4045566.

Dans un mode de réalisation, la teneur massique en gélifiant polyamide est comprise dans un intervalle allant de 0 à 10 % par rapport à la masse totale de la composition solaire.

Dans un mode de réalisation, la teneur massique en gélifiant polyamide est comprise dans un intervalle allant de 0 à 8 % par rapport à la masse totale de la composition solaire.

Dans un mode de réalisation, la teneur massique en gélifiant polyamide est comprise dans un intervalle allant de 0 à 6 % par rapport à la masse totale de la composition solaire.

Dans un mode de réalisation, la teneur massique en gélifiant polyamide est comprise dans un intervalle allant de 1 à 10 % par rapport à la masse totale de la composition solaire.

Dans un mode de réalisation, la teneur massique en gélifiant polyamide est comprise dans un intervalle allant de 1 à 8 % par rapport à la masse totale de la composition solaire.

Dans un mode de réalisation, la teneur massique en gélifiant polyamide est comprise dans un intervalle allant de 1 à 6 % par rapport à la masse totale de la composition solaire.

Dans un mode de réalisation, la teneur massique en gélifiant polyamide est comprise dans un intervalle allant de 2 à 10 % par rapport à la masse totale de la composition solaire.

Dans un mode de réalisation, la teneur massique en gélifiant polyamide est comprise dans un intervalle allant de 2 à 8 % par rapport à la masse totale de la composition solaire.

Dans un mode de réalisation, la teneur massique en gélifiant polyamide est comprise dans un intervalle allant de 2 à 6 % par rapport à la masse totale de la composition solaire.

Dans un mode de réalisation, la formulation solaire selon l'invention est caractérisée en ce que le gélifiant polyamide est un copolymère d'acide sébacique et d'huile de ricin hydrogénée commercialisée sous la dénomination ESTOGEL^{®} GREEN.

Dans un mode de réalisation, la teneur massique en ESTOGEL^{®} GREEN est comprise dans un intervalle allant de 0 à 10 % par rapport à la masse totale de la composition solaire.

Dans un mode de réalisation, la teneur massique en ESTOGEL^{®} GREEN est comprise dans un intervalle allant de 0 à 8 % par rapport à la masse totale de la composition solaire.

Dans un mode de réalisation, la teneur massique en ESTOGEL^{®} GREEN est comprise dans un intervalle allant de 0 à 6 % par rapport à la masse totale de la composition solaire.

Dans un mode de réalisation, la teneur massique en ESTOGEL^{®} GREEN est comprise dans un intervalle allant de 1 à 10 % par rapport à la masse totale de la composition solaire.

Dans un mode de réalisation, la teneur massique en ESTOGEL^{®} GREEN est comprise dans un intervalle allant de 1 à 8 % par rapport à la masse totale de la composition solaire.

Dans un mode de réalisation, la teneur massique en ESTOGEL^{®} GREEN est comprise dans un intervalle allant de 1 à 6 % par rapport à la masse totale de la composition solaire.

Dans un mode de réalisation, la teneur massique en ESTOGEL^{®} GREEN est comprise dans un intervalle allant de 2 à 10 % par rapport à la masse totale de la composition solaire.

Dans un mode de réalisation, la teneur massique en ESTOGEL^{®} GREEN est comprise dans un intervalle allant de 2 à 8 % par rapport à la masse totale de la composition solaire.

Dans un mode de réalisation, la teneur massique en ESTOGEL^{®} GREEN est comprise dans un intervalle allant de 2 à 6 % par rapport à la masse totale de la composition solaire.

Dans un mode de réalisation, la formulation solaire selon l'invention est caractérisée en ce que le gélifiant polyester est le trihydroxystéarin.

Dans un mode de réalisation, la teneur massique en trihydroxystéarin est comprise dans un intervalle allant de 0 à 5 % par rapport à la masse totale de la composition solaire.

Dans un mode de réalisation, la teneur massique en trihydroxystéarin est comprise dans un intervalle allant de 0 à 4 % par rapport à la masse totale de la composition solaire.

Dans un mode de réalisation, la teneur massique en trihydroxystéarin est comprise dans un intervalle allant de 0 à 3 % par rapport à la masse totale de la composition solaire.

Dans un mode de réalisation, la teneur massique en trihydroxystéarin est comprise dans un intervalle allant de 0 à 2 % par rapport à la masse totale de la composition solaire.

Dans un mode de réalisation, la teneur massique en trihydroxystéarin est comprise dans un intervalle allant de 0,25 à 5 % par rapport à la masse totale de la composition solaire.

Dans un mode de réalisation, la teneur massique en trihydroxystéarin est comprise dans un intervalle allant de 0,25 à 4 % par rapport à la masse totale de la composition solaire.

Dans un mode de réalisation, la teneur massique en trihydroxystéarin est comprise dans un intervalle allant de 0,25 à 3 % par rapport à la masse totale de la composition solaire.

Dans un mode de réalisation, la teneur massique en trihydroxystéarin est comprise dans un intervalle allant de 0,25 à 2 % par rapport à la masse totale de la composition solaire.

Dans un mode de réalisation, la teneur massique en trihydroxystéarin est comprise dans un intervalle allant de 0,5 à 5 % par rapport à la masse totale de la composition solaire.

Dans un mode de réalisation, la teneur massique en trihydroxystéarin est comprise dans un intervalle allant de 0,5 à 4 % par rapport à la masse totale de la composition solaire.

Dans un mode de réalisation, la teneur massique en trihydroxystéarin est comprise dans un intervalle allant de 0,5 à 3 % par rapport à la masse totale de la composition solaire.

Dans un mode de réalisation, la teneur massique en trihydroxystéarin est comprise dans un intervalle allant de 0,5 à 2 % par rapport à la masse totale de la composition solaire.

Dans un mode de réalisation, la formulation solaire selon l'invention comprend en outre au moins un agent gélifiant pulvérulent.

Dans un mode de réalisation, la formulation solaire selon l'invention est caractérisée en ce que le gélifiant pulvérulent est le SILICA (INCI).

Dans un mode de réalisation, la teneur massique en SILICA (INCI) est comprise dans un intervalle allant de 0 à 10 % par rapport à la masse totale de la composition solaire.

Dans un mode de réalisation, la teneur massique en SILICA (INCI) est comprise dans un intervalle allant de 0 à 8 % par rapport à la masse totale de la composition solaire.

Dans un mode de réalisation, la teneur massique en SILICA (INCI) est comprise dans un intervalle allant de 0 à 6 % par rapport à la masse totale de la composition solaire.

Dans un mode de réalisation, la teneur massique en SILICA (INCI) est comprise dans un intervalle allant de 1 à 10 % par rapport à la masse totale de la composition solaire.

Dans un mode de réalisation, la teneur massique en SILICA (INCI) est comprise dans un intervalle allant de 1 à 8 % par rapport à la masse totale de la composition solaire.

Dans un mode de réalisation, la teneur massique en SILICA (INCI) est comprise dans un intervalle allant de 1 à 6 % par rapport à la masse totale de la composition solaire.

Dans un mode de réalisation, la teneur massique en SILICA (INCI) est comprise dans un intervalle allant de 2 à 10 % par rapport à la masse totale de la composition solaire.

Dans un mode de réalisation, la teneur massique en SILICA (INCI) est comprise dans un intervalle allant de 2 à 8 % par rapport à la masse totale de la composition solaire.

Dans un mode de réalisation, la teneur massique en SILICA (INCI) est comprise dans un intervalle allant de 2 à 6 % par rapport à la masse totale de la composition solaire.

Dans un mode de réalisation, la formulation solaire selon l'invention comprend au moins un agent antioxydant ou agent piégeur de radicaux libres.

Dans un mode de réalisation, la formulation solaire selon l'invention comprend au moins un agent antioxydant choisi dans le groupe constitué par l'acide ascorbique (vitamine C) et les dérivés de l'acide ascorbique.

Dans un mode de réalisation, la formulation solaire selon l'invention comprend au moins un agent antioxydant choisi dans le groupe constitué par l'acide ascorbique et ses sels, les esters d'acide gras de l'acide ascorbique, le phosphate d'ascorbyle de magnésium, le phosphate d'ascorbyle de sodium et le sorbate d'ascorbyle.

Dans un mode de réalisation, la formulation solaire selon l'invention comprend au moins un agent antioxydant choisi dans le groupe constitué par le tocophérol (vitamine E) et les dérivés du tocophérol choisis par exemple dans le groupe constitué du α-tocophérol, du β-tocophérol, du γ-tocophérol ou du δ-tocophérol.

Dans un mode de réalisation, la formulation solaire selon l'invention comprend au moins un agent antioxydant choisi dans le groupe constitué par le tocophérol, le sorbate de tocophérol, l'acétate de tocophérol et les esters du tocophérol.

Dans un mode de réalisation, la formulation solaire selon l'invention comprend au moins un agent antioxydant choisi dans le groupe constitué par les acides hydroxy-benzoïques butylés et leurs sels, les péroxydes incluant le peroxyde d'hydrogène et le perborate, les thioglycolates, les sels de persulfate, l'acide 6-hydroxy-2,5,7,8-tétraméthylchromane-2-carboxylique, l'acide gallique, les alkyles esters de l'acide gallique, le gallate de propyle, l'acide urique, les sels d'acide urique, les alkyles esters de l'acide urique, l'acide sorbique, les sels d'acide sorbique, l'acide lipoïque, les amines, la N,N-diéthylhydroxylamine, l'amino-1-guanidine, les composés sulfhydryles, la glutathione, l'acide dihydroxyfumarique, les sels d'acide dihydroxyfumarique, le pidolate de lysine, les acides aminés, la silymarine, la lysine, la 1-méthionine, la proline, la superoxide dismutase, les extraits d'olive, les extraits de thé, les polyphénols, la proanthocyanidine de l'écorce de pin, les caroténoïdes, les composés curcumin, le tétrahydrocurcumin, l'acide L-2-oxo-4-thiazolidine carboxylique (OCTA), la mélanine, les extraits de romarin et les extraits de peau/grains de raisin.

Dans un mode de réalisation, la formulation solaire selon l'invention est caractérisée en ce qu'elle comprend en outre au moins de l'huile végétale de Karanja ou l'un de ses constituants pour améliorer la protection solaire de la formulation selon l'invention.

Dans un mode de réalisation, le constituant d'huile végétale de Karanja est le pongamol.

Dans un mode de réalisation, la teneur massique en pongamol est comprise dans un intervalle allant de 0 à 3 % par rapport à la masse totale de la composition solaire.

Dans un mode de réalisation, la teneur massique en pongamol est comprise dans un intervalle allant de 0 à 2,5 % par rapport à la masse totale de la composition solaire.

Dans un mode de réalisation, la teneur massique en pongamol est comprise dans un intervalle allant de 0 à 2 % par rapport à la masse totale de la composition solaire.

Dans un mode de réalisation, la teneur massique en pongamol est comprise dans un intervalle allant de 0 à 1,5 % par rapport à la masse totale de la composition solaire.

Dans un mode de réalisation, la teneur massique en pongamol est comprise dans un intervalle allant de 0 à 1,25 % par rapport à la masse totale de la composition solaire.

Dans un mode de réalisation, la teneur massique en pongamol est comprise dans un intervalle allant de 0 à 1 % par rapport à la masse totale de la composition solaire.

Dans un mode de réalisation, la teneur massique en pongamol est comprise dans un intervalle allant de 0 à 0,75 % par rapport à la masse totale de la composition solaire.

Dans un mode de réalisation, la teneur massique en pongamol est comprise dans un intervalle allant de 0 à 0,5 % par rapport à la masse totale de la composition solaire.

Dans un mode de réalisation, la teneur massique en pongamol est comprise dans un intervalle allant de 0,05 à 3 % par rapport à la masse totale de la composition solaire.

Dans un mode de réalisation, la teneur massique en pongamol est comprise dans un intervalle allant de 0,05 à 2,5 % par rapport à la masse totale de la composition solaire.

Dans un mode de réalisation, la teneur massique en pongamol est comprise dans un intervalle allant de 0,05 à 2 % par rapport à la masse totale de la composition solaire.

Dans un mode de réalisation, la teneur massique en pongamol est comprise dans un intervalle allant de 0,05 à 1,5 % par rapport à la masse totale de la composition solaire.

Dans un mode de réalisation, la teneur massique en pongamol est comprise dans un intervalle allant de 0,05 à 1,25 % par rapport à la masse totale de la composition solaire.

Dans un mode de réalisation, la teneur massique en pongamol est comprise dans un intervalle allant de 0,05 à 1 % par rapport à la masse totale de la composition solaire.

Dans un mode de réalisation, la teneur massique en pongamol est comprise dans un intervalle allant de 0,05 à 0,75 % par rapport à la masse totale de la composition solaire.

Dans un mode de réalisation, la teneur massique en pongamol est comprise dans un intervalle allant de 0,05 à 0,5 % par rapport à la masse totale de la composition solaire.

Dans un mode de réalisation, la teneur massique en pongamol est comprise dans un intervalle allant de 0,1 à 3 % par rapport à la masse totale de la composition solaire.

Dans un mode de réalisation, la teneur massique en pongamol est comprise dans un intervalle allant de 0,1 à 2,5 % par rapport à la masse totale de la composition solaire.

Dans un mode de réalisation, la teneur massique en pongamol est comprise dans un intervalle allant de 0,1 à 2 % par rapport à la masse totale de la composition solaire.

Dans un mode de réalisation, la teneur massique en pongamol est comprise dans un intervalle allant de 0,1 à 1,5 % par rapport à la masse totale de la composition solaire.

Dans un mode de réalisation, la teneur massique en pongamol est comprise dans un intervalle allant de 0,1 à 1,25 % par rapport à la masse totale de la composition solaire.

Dans un mode de réalisation, la teneur massique en pongamol est comprise dans un intervalle allant de 0,1 à 1 % par rapport à la masse totale de la composition solaire.

Dans un mode de réalisation, la teneur massique en pongamol est comprise dans un intervalle allant de 0,1 à 0,75 % par rapport à la masse totale de la composition solaire.

Dans un mode de réalisation, la teneur massique en pongamol est comprise dans un intervalle allant de 0,1 à 0,5 % par rapport à la masse totale de la composition solaire.

Dans un mode de réalisation, la formulation solaire selon l'invention est caractérisée en ce qu'elle comprend en outre au moins un parfum.

Dans un mode de réalisation, le au moins un parfum est le monoï.

L'invention concerne également l'utilisation cosmétique de la composition solaire ci-dessus définie pour diminuer le photovieillisement de la peau saine et/ou diminuer les manifestations inesthétiques et/ou inconfortables de la peau saine liées à l'exposition au soleil, tels que des taches pigmentaires, des tiraillements, une sécheresse, des rougeurs.

L'invention concerne également la composition ci-dessus définie pour son utilisation thérapeutique et/ou dermatologique.

L'invention concerne également la composition ci-dessus définie son utilisation thérapeutique et/ou dermatologique pour protéger la peau, les muqueuses et/ou les phanères des rayonnements solaires.

L'invention concerne également le procédé de préparation d'une composition solaire définie ci-dessus comprenant les étapes suivantes :
- On dispose d'au moins un véhicule lipophile cosmétiquement et/ou dermatologiquement acceptable ;
- On chauffe ledit véhicule à une température au moins égale à 30 °C ;
- On ajoute l'oxyde de zinc non nanométrique, tel que précédemment défini ;
- On maintient sous agitation pendant au moins 15 min pendant la phase de refroidissement.

Dans un mode de réalisation des excipients complémentaires liposolubles sont ajoutés dans le véhicule avant la phase de chauffage ou pendant la phase de refroidissement.

Après refroidissement complet, la composition selon l'invention est conditionnée en flacons.

L'invention concerne également un spray solaire comprenant la composition solaire selon l'invention.

L'invention concerne également un lait solaire comprenant la composition solaire selon l'invention.

### Exemples de formulations solaires anhydres contenant uniquement de l'oxyde de Zinc.

### Exemple 1 : Protocole de fabrication des formulations solaires anhydres

1. Peser le mélange A et le faire chauffer à 55 °C sous agitation pendant 30 min ;
2. Préparer le mélange B. Faire chauffer l'huile à 75 °C. Une fois l'huile chaude, ajouter le Pongamol et homogénéiser pendant 15 min au barreau aimanté ;
3. Ajouter le mélange B dans A et maintenir à 55 °C pendant 10 min ;
4. Ajouter le filtre et homogénéiser pendant 10 min sous agitation ;
5. Couper la chauffe et maintenir l'agitation ;
6. A partir de 35 °C, ajouter le mélange D.

### Exemple 2 : Formule solaire avec un indice SPF50 sans gélifiant

**Tableau 1**

| Formule solaire SPF 50 sans gélifiant | | | |
|---|---|---|---|
| Mélange | Référence commerciale | Référence INCI | Pourcentage massique (%) |
| A | HV Fruit de la passion BIO | Passiflora Edulis Seed Oil | 1,00 |
| A | Lexfilm Sun Natural MB | Capryloyl Glycerin/Sebacic Acid Copolymer | 5,00 |
| A | Dry Touch | Dodecane | 5,64 |
| A | Caprylis sans palme | Caprylic/Capric Triglycéride | 7,20 |
| A | HV Sésame BIO désodorisé | Sesamum Indicum Seed Oil | 23,10 |
| A | Isoamyl Laurate | Isoamyl Laurate | 8,75 |
| A | Applecare PDS 300 | Caprylic/Capric Triglyceride, Polyhydroxystearic Acid, Isostearic Acid, Lecithin, Polyglyceryl-3 Polyricinoleate | 2,00 |
| B | Pongamol | Pongamol | 0,25 |
| B | Coco Silicone | Coco-Caprylate, Tocopherol, Helianthus Annuus Seed Oil | 4,00 |
| C | Xperse 102 | Zinc Oxide, Caprylic/Capric Triglycéride, Polyhydroxystearic Acid | 41,66 |
| D | Monoï santal pour solaire | Parfum | 1,20 |
| D | Vitamine E | Helianthus Annuus Seed Oil, Tocopherol | 0,20 |
| Total | | | 100,00 |

### Exemple 3 : Formule solaire avec un indice SPF50 contenant un gélifiant A

**Tableau 2**

| Formule solaire SPF 50 avec gélifiant A | | | |
|---|---|---|---|
| Mélange | Référence commerciale | Référence INCI | Pourcentage massique (%) |
| A | HV Fruit de la passion BIO | Passiflora Edulis Seed Oil | 1,00 |
| A | Isoamyl Laurate | Isoamyl Laurate | 8,75 |
| A | HV Sésame BIO désodorisé | Sesamum Indicum Seed Oil | 20,60 |
| A | Lexfilm Sun Natural MB | Capryloyl Glycerin/Sebacic Acid Copolymer | 5,00 |
| A | Dry Touch | Dodecane | 5,64 |
| A | Caprylis sans palme | Caprylic/Capric Triglycéride | 7,20 |
| A | Estogel Green | Hydrogenated Castor Oil/ Sebacic Acid Copolymer (gélifiant A) | 2,50 |
| A | Applecare PDS 300 | Caprylic/Capric Triglyceride, Polyhydroxystearic Acid, Isostearic Acid, Lecithin, Polyglyceryl-3 Polyricinoleate | 2,00 |
| B | Coco Silicone | Coco-Caprylate, Tocopherol, Helianthus Annuus Seed Oil | 4,00 |
| B | Pongamol | Pongamol | 0,25 |
| C | Xperse 102 | Zinc Oxide, Caprylic/Capric Triglycéride, Polyhydroxystearic Acid | 41,66 |
| D | Monoï santal pour solaire | Parfum | 1,20 |
| D | Vitamine E | Helianthus Annuus Seed Oil, Tocopherol | 0,20 |
| Total | | | 100,00 |

### Exemple 4 : Formule solaire avec un indice SPF50 contenant un gélifiant B

**Tableau 3**

| Formule solaire SPF 50 / gélifiant B | | | |
|---|---|---|---|
| Mélange | Référence commerciale | Référence INCI | Pourcentage massique (%) |
| A | Isoamyl Laurate | Isoamyl Laurate | 6,00 |
| A | Caprylis sans palme | Caprylic/Capric Triglycéride | 14,30 |
| A | Dry Touch | Dodecane | 9,39 |
| A | Trihydroxystearin | Trihydroxystearin (gélifiant B) | 1,40 |
| A | HV fruit de la passion BIO | Passiflora Edulis Seed Oil | 3,00 |
| A | Applecare PDS 300 | Caprylic/Capric Triglycéride, Polyhydroxystearic Acid, Isostearic Acid, Lecithin, Polyglyceryl-3 Polyricinoleate | 2,00 |
| A | HV Carthame BIO | Carthamus Tinctorius Seed Oil | 17,00 |
| B | Caprylis sans palme | Caprylic/Capric Triglycéride | 4,00 |
| B | Pongamo) | Pongamol | 0,25 |
| C | Xperse 102 | Zinc Oxide, Caprylic/Capric Triglyceride, Polyhydroxystearic Acid | 41,66 |
| D | Vitamine E | Helianthus Annuus Seed Oil, Tocopherol | 0,20 |
| D | Coconut Water RT60349 | Cocos Nucifera Water | 0,80 |
| Total | | | 100,00 |

### Exemple 5 : Formule solaire avec un indice SPF30 sans gélifiant

**Tableau 4**

| Formule solaire SPF 30 | | | |
|---|---|---|---|
| Mélange | Référence commerciale | Référence INCI | Pourcentage massique (%) |
| A | Isoamyl Laurate | Isoamyl Laurate | 8,75 |
| A | HV Sésame BIO désodorisé | Sesamum Indicum Seed Oil | 24,76 |
| A | Lexfilm Sun Natural MB | Capryloyl Glycerin/Sebacic Acid Copolymer | 5,00 |
| A | Caprylis sans palme | Caprylic/Capric Triglycéride | 14,20 |
| A | HV fruit de la passion BIO | Passiflora Edulis Seed Oil | 1,00 |
| A | Dry Touch | Dodecane | 5,64 |
| B | Coco Silicone | Coco-Caprylate, Tocopherol, Helianthus Annuus Seed Oil | 4,00 |
| B | Pongamol | Pongamol | 0,25 |
| C | Xperse 102 | Zinc Oxide, Caprylic/Capric Triglycéride, Polyhydroxystearic Acid | 35,00 |
| D | Monoï santal pour solaire | Parfum | 1,20 |
| D | Vitamine E | Helianthus Annuus Seed Oil, Tocopherol | 0,20 |
| Total | | | 100,00 |

### Exemple 6 : Formule solaire avec un indice SPF30 contenant un gélifiant A

**Tableau 5**

| Formule solaire SPF 30 / gélifiant A | | | |
|---|---|---|---|
| Mélange | Référence commerciale | Référence INCI | Pourcentage massique (%) |
| A | Isoamyl Laurate | Isoamyl Laurate | 8,75 |
| A | Dry Touch | Dodecane | 5,64 |
| A | HV Sésame BIO désodorisé | Sesamum Indicum Seed Oil | 21,76 |
| A | Lexfilm Sun Natural MB | Capryloyl Glycerin/Sebacic Acid Copolymer | 5,00 |
| A | Caprylis sans palme | Caprylic/Capric Triglycéride | 14,20 |
| A | Estogel Green | Hydrogenated Castor Oil/ Sebacic Acid Copolymer (gélifiant A) | 3,00 |
| A | HV fruit de la passion BIO | Passiflora Edulis Seed Oil | 1,00 |
| B | Coco Silicone | Coco-Caprylate, Tocopherol, Helianthus Annuus Seed Oil | 4,00 |
| B | Pongamol | Pongamol | 0,25 |
| C | Xperse 102 | Zinc Oxide, Caprylic/Capric Triglycéride, Polyhydroxystearic Acid | 35,00 |
| D | Vitamine E | Helianthus Annuus Seed Oil, Tocopherol | 0,20 |
| D | Monoï santal pour solaire | Parfum | 1,20 |
| Total | | | 100,00 |

### Exemple 7 : Formule solaire avec un indice SPF30 contenant un gélifiant B

**Tableau 6**

| Formule solaire SPF 30 / gélifiant B | | | |
|---|---|---|---|
| Mélange | Référence commerciale | Référence INCI | Pourcentage massique (%) |
| A | Trihydroxystearin | Trihydroxystearin (gélifiant B) | 1,20 |
| A | HV fruit de la passion BIO | Passiflora Edulis Seed Oil | 3,00 |
| A | Dry Touch | Dodecane | 16,35 |
| A | Caprylis sans palme | Caprylic/Capric Triglycéride | 16,20 |
| A | HV Carthame BIO | Carthamus Tinctorius Seed Oil | 17,00 |
| A | Gosulin IL MB | Isoamyl Laurate/lsoamyl Cocoate | 10,00 |
| B | Assure + | Pongamol | 0,25 |
| B | Caprylis sans palme | Caprylic/Capric Triglycéride | 10,00 |
| C | Xperse 102 | Zinc Oxide, Caprylic/Capric Triglycéride, Polyhydroxystearic Acid | 25,00 |
| D | Vitamine E | Helianthus Annuus Seed Oil, Tocopherol | 0,20 |
| D | Coconut Water RT60349 | Cocos Nucifera Water | 0,80 |
| Total | | | 100,00 |

### Exemple 8 : Formule solaire avec un indice SPF50 contenant un gélifiant C

Protocole de fabrication de la formulation :
1. Peser le mélange A et le faire chauffer à 55 °C sous agitation pendant 30 min ;
2. Préparer le mélange B. Faire chauffer l'huile à 75 °C. Une fois l'huile chaude, ajouter le Pongamol et homogénéiser pendant 15 min au barreau aimanté ;
3. Ajouter le mélange B dans A et maintenir sous agitation à 55 °C pendant 10 min ;
4. Ajouter le filtre et homogénéiser pendant 10 min sous agitation ;
5. Ajouter le gélifiant C : Aerosil 200 sous agitations ;
6. Couper la chauffe et maintenir l'agitation ;
7. A partir de 35 °C, ajouter le mélange D sous agitation.

**Tableau 7**

| Lait Solaire SPF50 / gélifiant | | | |
|---|---|---|---|
| Mélange | Référence commerciale | Référence INCI | Pourcentage massique (%) |
| A | Dry Touch | Dodecane | 9,39 |
| A | Applecare PDS 300 | Caprylic/Capric Triglycéride, Polyhydroxystearic Acid, Isostearic Acid, Lecithin, Polyglyceryl-3 Polyricinoleate | 2,00 |
| A | Huile Végétale Sésame BIO désodorisé | Sesamum Indicum Seed Oil | 19,00 |
| A | Dermofeel Sensolv MB | Isoamyl Laurate | 6,00 |
| A | Huile Végétale Fruit de la passion BIO | Passiflora Edulis Seed Oil | 1,00 |
| A | Caprylis sans palme | Caprylic/Capric Triglycéride | 11,70 |
| B | Assure + | Pongamol | 0,25 |
| B | Coco silicone | Coco-Caprylate, Tocopherol, Helianthus Annuus Seed Oil | 4,00 |
| C | Xperse 102 | Zinc Oxide, Caprylic/Capric Triglyceride, Polyhydroxystearic Acid | 41,66 |
| D | Aerosil 200 | Silica | 4,00 |
| D | Vitamine E | Helianthus Annuus Seed Oil, Tocopherol | 0,20 |
| D | Coconut Water RT60349 | Cocos Nucifera Water | 0,80 |
| Total | | | 100,00 |

### Exemple 9 : Lait solaire avec un indice SPF30

Protocole de fabrication de la formulation d'un lait solaire SPF30:
1. Peser le mélange A, contenant le filtre, et le faire chauffer à 80 °C sous agitation lente pendant 45 min ;
2. Vérifier l'homogénéisation du mélange ;
3. Préparer le mélange B. Faire chauffer l'huile à 85 °C. Une fois l'huile chaude, ajouter le Pongamol et homogénéiser pendant 15 min au barreau aimanté ;
4. Ajouter le mélange B, à 85 °C, dans A et agiter pendant 5 min à 85 °C, puis refroidir le mélange jusqu'à 40 °C sous faible agitation ;
5. Refroidir à 35 °C, ajouter le mélange C et homogénéiser le tout pendant 2 min.

**Tableau 8**

| Lait Solaire SPF30 (E47) | | | |
|---|---|---|---|
| Mélange | Référence commerciale | Référence INCI | Pourcentage massique (%) |
| A | Dry Touch RSPO | Dodecane | 8,64 |
| A | Huile Végétale Fruit de la passion BIO | Passiflora Edulis Seed Oil | 1,00 |
| A | Solaveil MZ3 300 | Zinc Oxide, Caprylic Capric Triglycéride, Polyhydroxystearic Acid | 38,00 |
| A | Estogel Green | Hydrogenated Castor Oil/Sebacic Acid Copolymer | 3,50 |
| A | Applecare PDS 300 RSPO | Caprylic/Capric Triglyceride, Polyhydroxystearic Acid, Isostearic Acid, Lecithin, Polyglyceryl-3 Polyricinoleate | 2,00 |
| A | Lexfilm Sun Natural MB RSPO | Capryloyl Glycerin/Sebacic Acid Copolymer | 5,00 |
| A | Caprylis sans palme | Caprylic/Capric Triglyceride | 6,86 |
| A | Isoamyl laurate RSPO | Isoamyl Laurate | 8,75 |
| A | Huile Végétale Sésame BIO désodorisé | Sesamum Indicum Seed Oil | 20,6 |
| B | Cetiol C5 C | Coco-Caprylate, Tocopherol | 4,00 |
| B | Pongamol | Pongamol | 0,25 |
| C | Monoï santal pour solaire | Parfum | 1,20 |
| C | Vitamine E | Helianthus Annuus Seed Oil, Tocopherol | 0,20 |
| Total | | | 100,00 |

### Exemple 10 : Lait solaire avec un indice SPF30

Protocole de fabrication de la formulation d'un lait solaire SPF30 :
1. Peser le mélange A, contenant le filtre, et le faire chauffer à 80 °C sous agitation lente pendant 45 min ;
2. Vérifier l'homogénéisation du mélange ;
3. Préparer le mélange B. Faire chauffer l'huile à 85 °C. Une fois l'huile chaude, ajouter le Pongamol et homogénéiser pendant 15 min au barreau aimanté ;
4. Ajouter le mélange B, à 85 °C, dans A et agiter pendant 5 min à 85 °C, puis refroidir le mélange jusqu'à 40 °C sous faible agitation ;
5. Refroidir à 35 °C, ajouter le mélange C et homogénéiser le tout pendant 2 min.

**Tableau 9**

| Lait Solaire SPF30 (E20) | | | |
|---|---|---|---|
| Mélange | Référence commerciale | Référence INCI | Pourcentage massique (%) |
| A | Isoamyl laurate RSPO | Isoamyl Laurate | 8,75 |
| A | Solaveil MZ3 300 | Zinc Oxide, Caprylic Capric Triglyceride, Polyhydroxystearic Acid | 35,00 |
| A | Dry Touch RSPO | Dodecane | 5,64 |
| A | Huile Végétale Sésame BIO désodorisé | Sesamum Indicum Seed Oil | 21,76 |
| A | Lexfilm Sun Natural MB RSPO | Capryloyl Glycerin/Sebacic Acid Copolymer | 5,00 |
| A | Caprylis sans palme | Caprylic/Capric Triglyceride | 14,20 |
| A | Estogel Green | Hydrogenated Castor Oil/Sebacic Acid Copolymer | 3,00 |
| A | Huile Végétale Fruit de la passion BIO | Passiflore Edulis Seed Oil | 1,00 |
| B | Cetiol C5 C | Coco-Caprylate, Tocopherol | 4,00 |
| B | Pongamol | Pongamol | 0,25 |
| C | Vitamine E | Helianthus Annuus Seed Oil, Tocopherol | 0,20 |
| C | Monoï santal pour solaire | Parfum | 1,20 |
| Total | | | 100,00 |

## Revendications

1. Composition solaire exempte d'eau adaptée à un usage topique, comprenant de l'oxyde de zinc qui présente une D50 supérieure ou égale à 200 nm et une D1 supérieure ou égale à 105 nm-en suspension dans un véhicule lipophile cosmétiquement et/ou dermatologiquement acceptable, ladite composition solaire ne comprenant pas d'oxyde de titane, **caractérisée en ce que** la teneur massique en oxyde de zinc est comprise dans un intervalle allant de 10 à 40 % par rapport à la masse totale de la composition solaire,
**en ce que** la composition solaire est exempte de cire ayant une température de fusion comprise dans un intervalle allant de 35 à 72 °C et/ou de dérivés siliconés,
et **en ce que** le véhicule lipophile cosmétiquement et/ou dermatologiquement acceptable comprend un excipient choisi dans le groupe des alcanes.

2. Composition solaire selon la revendication 1 , **caractérisée en ce qu'**elle est exempte de polyvinylpyrrolidone et/ou de ses dérivés.

3. Composition solaire selon la revendication 1, **caractérisée en ce qu'**elle est exempte de filtres solaires organiques synthétiques.

4. Composition solaire selon l'une quelconque des revendications précédentes, **caractérisée en ce qu'**elle présente un indice de protection SPF supérieur ou égale à 20.

5. Composition solaire selon l'une quelconque des revendications précédentes, **caractérisée en ce que** le véhicule lipophile cosmétiquement et/ou dermatologiquement acceptable comprend en outre au moins un excipient choisi dans le groupe comprenant le caprilyc/capric triglycéride, l'acide polyhydroxystéarique.

6. Composition solaire selon la revendication 5, **caractérisée en ce que** la teneur massique en caprilyc/capric triglycéride est comprise dans un intervalle allant de 19 à 35 % par rapport à la masse totale de la composition solaire.

7. Composition solaire selon l'une quelconque des revendications précédentes, **caractérisée en ce que** l'excipient choisi dans le groupe des alcanes est du dodécane.

8. Composition solaire selon l'une quelconque des revendications précédentes, **caractérisée en ce qu'**elle comprend en outre au du pongamol.

9. Composition solaire selon la revendication 8, **caractérisée en ce que** la teneur massique en pongamol est comprise dans un intervalle allant de 0 à 3 % par rapport à la masse totale de la composition solaire.

10. Composition solaire selon l'une quelconque des revendications précédentes, **caractérisée en ce qu'**elle comprend en outre un copolymère filmogène.

11. Composition solaire selon la revendication 10, **caractérisée en ce que** la teneur massique en copolymère filmogène est comprise dans un intervalle allant de 0 à 15 % par rapport à la masse totale de la composition solaire.

12. Composition solaire selon les revendications 10 et 11, **caractérisée en ce que** le copolymère filmogène est le Capryloyl Glycerin/Sebacic Acid Copolymer.

13. Composition solaire selon l'une quelconque des revendications précédentes, **caractérisée en ce qu'**elle comprend en outre un gélifiant polymérique choisi dans le groupe comprenant : les polyesters et leurs dérivés, les résines de polyamides, les polyuréthanes et/ou leurs mélanges.

14. Composition solaire selon la revendication 13, **caractérisée en ce que** la teneur massique en gélifiant polymérique est comprise dans un intervalle allant de 0 à 10 % par rapport à la masse totale de la composition solaire.

15. Composition solaire selon l'une quelconque des revendications 1 à 14, pour son utilisation thérapeutique et/ou dermatologique.

16. Composition solaire pour pour son utilisation thérapeutique et/ou dermatologique selon la revendication 15, pour protéger la peau, les muqueuses et/ou les phanères des rayonnements solaires.

17. Procédé de préparation d'une composition solaire l'une quelconque des revendications précédentes 1 à 16 comprenant les étapes suivantes :
a) On dispose d'au moins un véhicule cosmétiquement et/ou dermatologiquement acceptable ;
b) On chauffe ledit véhicule à une température au moins égale à 30°C ;
c) On ajoute l'oxyde de zincprésentant une D50 supérieure ou égale à 200 nm et une D1 supérieure ou égale à 105 nm;
d) On maintient sous agitation pendant au moins 15 mn pendant la phase de refroidissement.
